# EUROPEAN PATENT APPLICATION

(11) **EP 3 011 983 A1**
(43) Date of publication of application: **27.04.2016**
(21) Application number: 13887227.0
(22) Date of filing: 22.11.2013
(51) Int. Cl.: A61M 1/00, A61M 3/00, A61M 39/00

(54) **MULTIFUNCTIONAL FLUSHING AND DRAWING CATHETER**

(30) Priority: 21.06.2013 CN 201310247469; 23.10.2013 CN 201310501972
(71) Applicant: Qian, Jianmin, Wuxi, Jiangsu 214192 (CN)
(72) Inventor: QIAN, Jianmin, Wuxi, Jiangsu 214192 (CN); HUANG, Xiujuan, Wuxi, Jiangsu 214192 (CN); QIAN, Wei, Wuxi, Jiangsu 214192 (CN)
(74) Representative: Engelhard, Markus
(86) International application number: PCT/CN2013/087663
(87) International publication number: WO 2014/201804

(57) **Abstract**

A multifunctional suction irrigator includes a handle (1) and a tube portion (2), where an end portion of one end of the tube portion (2) is provided with a suction and irrigation channel (3) and a lighting channel (4), a tail portion of the handle (1) is provided with a negative pressure joint (5) and an irrigation joint (6), an irrigation tip and a negative pressure suction tip are installed in the suction and irrigation channel (3), the negative pressure suction tip is in communication with the negative pressure joint (5) through a negative pressure tube (7), and the irrigation tip is in communication with the irrigation joint (6) through an irrigation tube (8); a roller-type negative pressure volume controller (11) for controlling a negative pressure value of the negative pressure tube (7) and a roller-type irrigation volume controller (12) for controlling an irrigation volume of the irrigation tube (8) are installed on the handle (1); and an LED floodlight (9) is installed in the lighting channel (4), and electrically connected to a battery (13) installed in the handle (1) by using a conducting wire (10), and a push-button switch for controlling on/off of the LED floodlight (9) is installed on the handle (1). The multifunctional suction irrigator is convenient and labor-saving in operation, simple in structure, and convenient in use.

## Description

### BACKGROUND

### Technical Field

The present invention relates to a medical treatment instrument, particularly to an operation sucker for irrigating and sucking a wound surface in an operation process, and specifically to a multifunctional suction irrigator.

### Related Art

It is well-known that, when a surgical operation is performed, a surgical field is required to be kept clear, so that a surgeon can perform a precise operation on an operating position, and therefore during an operation, it is needed to perform negative pressure suction on continuously exuded blood, an excised tissue and tissue body fluid and continuously irrigate a bleeding position by using physiological saline to ensure a clear surgical field. Therefore, when negative pressure suction is performed during an operation, not only a suction tip having a negative pressure suction function needs to be used, but also the suction tip needs to be capable of performing irrigation with physiological saline at the same time of suction. Moreover, in a case of a lighting blind angle or insufficient illumination of an operating shadowless lamp, an additional auxiliary apparatus is needed to perform lighting. However, when tissue body fluid on an operating position such as exuded blood is severe, an irrigation tip further needs to be used for continuously performing intra-operative irrigation and synchronous negative pressure suction. In a case in which the three actions are performed simultaneously, the burden on the surgeon is greatly increased, and work efficiency and accuracy of the doctor during the operation are greatly affected. Therefore, a multifunctional operation sucker integrated with negative pressure suction and physiological saline irrigation is invented. For example, Chinese Patent No. ZL2011100538415 previously applied by the applicant discloses a bloclcing-proof multifunctional operation sucker that relatively well resolves the foregoing problem. However, the applicant finds during use that this operating suction irrigator has problems such as a complex structure and an inconvenient operation. Particularly, a negative pressure controller and an irrigation volume controller are complex in structure, a needed operation strength is large, a negative pressure value and an irrigation volume are adjusted inconveniently, an operator needs to press a corresponding switch by using a large strength before irrigation and suction can be implemented, and the operator is very easily tired. Additionally, a tip portion of each conventional sucker is a perpendicular plane, and an end surface of the tip portion is perpendicular to a pipeline of a suction tip. However, in an actual operation, once a relatively large blood block is encountered, the blood block cannot be sucked due to effect of the tube diameter of the negative pressure suction tip. In this case, manual clearing needs to be used, and a tool needs to be replaced before the blood block can be cleared to find a bleeding point. However, the conventional sucker cannot be directly used in replace of the tool due to the perpendicular end surface, which causes an inconvenient operation and a prolonged operation time. Furthermore, because the patent uses a light guiding material as a lighting source, a problem of insufficient illuminance also exists. Moreover, when a bleeding position is treated during specific use, because of the defect of the structure of the end portion, both irrigation and suction need to be aligned with the bleeding position. Because of existence of a high-strength negative pressure, when negative pressure suction is performed, a blood vessel injury of a bleeding position of a tissue is aggravated because of the action of the negative pressure, the bleeding volume of the bleeding position is increased, which adversely causes a bad irrigation effect and an unclear visual field, increases the bleeding volume, and increases an operation risk, for which there is currently no good solution, so as to affect popularization and application of the type of operation instruments.

### SUMMARY

For a problem that a conventional negative pressure suction tip with an irrigation function needs a large operation strength during use, adjustment of a negative pressure value and an irrigation volume is inconvenient and a bleeding volume is increased during use due to an action of a negative pressure on a bleeding position, an objective of the present invention is to design a multifunctional suction irrigator that is convenient and labor-saving in operation and convenient in adjustment of an irrigation volume and a negative pressure value and enables a negative pressure suction hole to be away from a bleeding point to only suck a liquid flowing out after being irrigated through an irrigation hole or a liquid around the bleeding point.

A first technical solution of the present invention is:
A multifunctional suction irrigator includes a handle 1 and a tube portion 2, where an end portion of one end, of the tube portion 2, not connected to the handle 1 is provided with a suction and irrigation channel 3 and a lighting channel 4, a tail portion of the handle 1 is provided with a negative pressure joint 5 and an irrigation joint 6, an irrigation tip and a negative pressure suction tip are installed in the suction and irrigation channel 3, the negative pressure suction tip is in communication with the negative pressure joint 5 by passing through the tube portion and a negative pressure tube 7 of a cavity of the handle, and the irrigation tip is in communication with the irrigation joint 6 by passing through the tube portion and an irrigation tube 8 of the cavity of the handle; a roller-type negative pressure volume controller 11 for controlling a negative pressure value of the negative pressure tube 7 and a roller-type irrigation volume controller 12 for controlling an irrigation volume of the irrigation tube 8 are installed on the handle 1; and an LED floodlight 9 is installed in the lighting channel 4, and electrically connected to a battery 13 installed in the handle 1 by using a conducting wire 10, and a push-button switch for controlling on/off of the LED floodlight 9 is installed on the handle 1.

An angle between an end surface of the tube portion 2 and an axis of the tube portion is less than 90 degrees so as to conveniently scrape and clean an operating position by using the end surface, and conveniently observe a bleeding position.

The irrigation tip and the negative pressure suction tip in the suction and irrigation channel 3 are positioned therein by using solidification glue, or fixed by using a blocking sheet.

An uppermost portion of the handle 1 is connected to the tube portion 2 by using two smoothly transitional opposite arc segments, so as to further improve convenience of a surgical field and operations, where an opening of an arc segment close to the tube portion 2 is upward.

The handle 1 and the tube portion are made of a transparent material, so as to conveniently observe a blocking state of an internal channel, or not made of a transparent material.

A second technical solution of the present invention is:
A multifunctional suction irrigator includes a handle 1 and a tube portion 2, where an end portion of one end, of the tube portion 2, not connected to the handle 1 is provided with a suction and irrigation channel 3 and a lighting channel 4, a tail portion of the handle 1 is provided with a negative pressure joint 5 and an irrigation joint 6, an irrigation tip and a negative pressure suction tip are installed in the suction and irrigation channel 3, the negative pressure suction tip is in communication with the negative pressure joint 5 by passing through the tube portion and a negative pressure tube 7 of a cavity of the handle, and the irrigation tip is in communication with the irrigation joint 6 by passing through the tube portion and an irrigation tube 8 of the cavity of the handle; a roller-type negative pressure volume controller 11 for controlling a negative pressure value of the negative pressure tube 7 and a roller-type irrigation volume controller 12 for controlling an irrigation volume of the irrigation tube 8 are installed on the handle 1; and an irrigation outlet 3' in the suction and irrigation channel 3 is located at an upper portion of an inclined surface 7' of the end portion of the tube portion 2, and a negative pressure suction hole 4' of the negative pressure joint 5 is mainly located on a bottom surface 8' adjacent to the inclined surface 7' of the end portion of the tube portion 2.
a channel in the tube portion 2 is formed by a tube installed therein or a separate cavity formed in a tube; and the tube portion 2 is further provided with a lighting channel, and a lamp of the lighting channel is installed at a side of the irrigation outlet 3, or the tube portion 2 is provided with no lighting channel.

An adjustable switch for controlling a flow volume of a negative pressure channel and an adjustable switch for controlling a flow volume of an irrigation channel are disposed on the handle 1, and can control a water flow volume of irrigation and a flow volume of negative pressure suction respectively.
a negative pressure supplement suction hole 9' is also provided on the inclined surface 7' of the end portion of the tube portion 2, several lateral negative pressure suction holes 10' are provided on hole walls of two sides or one side of the negative pressure suction hole 4', the negative pressure suction hole 4', the negative pressure supplement suction hole 9' and the lateral negative pressure suction holes 10' are in communication with each other, and a highest location of the lateral negative pressure suction holes 10' does not exceed a horizontal surface of a central axis of the tube portion 2, and the aperture of the supplement suction hole should be obviously less than the aperture of the suction hole 4'.

A slant angle α of the inclined surface 7' is less than 70 degrees; and the inclined surface 7' is formed by two segments of inclined surfaces with different slant angles, and a slant angle of an upper inclined surface is greater than a slant angle of a lower inclined surface.

Beneficial effects of the present invention:
1. In the present invention, a roller-type transfusion volume adjuster for adjusting a transfusion speed used in transfusion is inventively used for adjusting a negative pressure value and an irrigation volume, the negative pressure value or irrigation volume can be adjusted by pushing a roller, and the present invention has advantages such as a simple structure, low costs, and convenient and labor-saving operations.
2. In the present invention, a button battery and an LED lamp are used for lighting, the present invention is convenient in manufacturing and installation, small in power supply volume, secure and reliable, and strong in illuminance, and experiments prove that use requirements can be completely satisfied, and an ideal lighting effect may be obtained.
3. In the present invention, an end surface of a suction irrigator portion is inventively designed into an inclined surface, the tube portion may be used by an operator as a clearing tool to clear a large blood block of a bleeding position by directly using the inclined surface, replacement with another operation instrument is not needed, an application range of an irrigator is further expanded, and a small reform generates a large effect.
4. In the present invention, a handle and the tube portion are re-designed, a direct connection manner is changed, and it is proved by using an experiment that, compared with a conventional structure, the present invention can obtain a better surgical field, better conform to human-machine engineering requirements, and be convenient in operation.
5. The present invention resolves a problem that cannot be resolved in a long term, where during negative pressure suction, blood vessel injury of the bleeding position may be aggravated and the bleeding volume is increased, so as to cause a bad irrigation and suction effect.
6. The present invention really implements a function of irrigation while suction, and can clear bleeding, an exuded liquid or another liquid of the operating position in time and keep a clear surgical field without aggravating the tissue injury.

### BRIEF DESCRIPTION OF THE DRAWINGS

FIG. 1 is a first schematic structural diagram of the present invention;
FIG. 2 is a rear view of FIG. 1;
FIG. 3 is a schematic three-dimensional structural outside view of the present invention;
FIG. 4 is a second schematic structural diagram of the present invention; and
FIG. 5 is a schematic enlarged diagram of an internal structure of an end portion of FIG. 4.

### DETAILED DESCRIPTION

The present invention is further described with reference to accompanying drawings and embodiments below.

### Embodiment 1

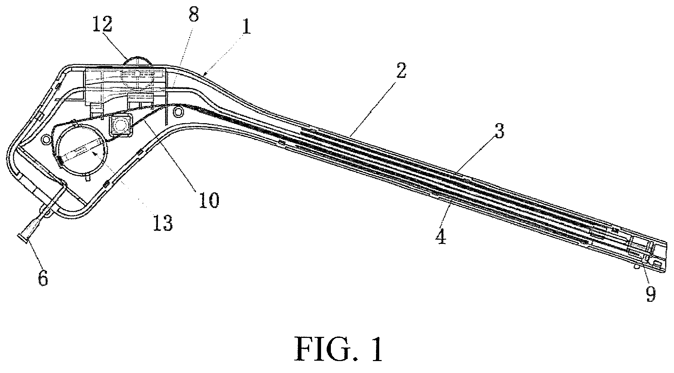

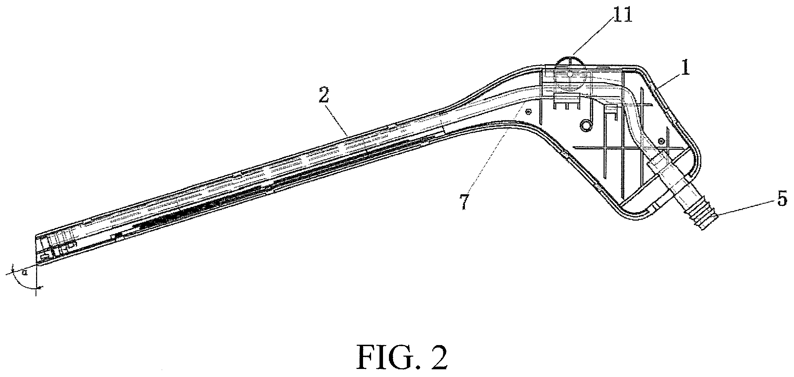

As shown in FIG. 1 to FIG.3:
A multifunctional suction irrigator is formed by connecting a handle 1 and a tube portion 2 (which may be a straight tube or crooked tube) integrally, the handle 1 and the tube portion 2 may be injection molded by using a transparent material (or non-transparent material) to form a snap-fit casing structure, and when the handle 1 and the tube portion 2 are made of a transparent material, it is convenient to observe a blocking state of an internal channel during use. The handle 1 is connected to the tube portion 2 by using two smoothly transitional opposite arc segments, so as to further improve convenience of a surgical field and operations, where an opening of an arc segment close to the tube portion 2 is upward. For convenience of scraping and cleaning an operating position by using an end surface, and for convenience of observing a bleeding position, during specific implementation, an angle α between the end surface of the tube portion 2 and an axis of the tube portion should be designed to be less than 90 degrees, optimally about 60 degrees, as shown in FIG. 1 and FIG. 2. An end portion of one end, of the tube portion 2, not connected to the handle 1 is provided with a suction and irrigation channel 3 and a lighting channel 4, a tail portion of the handle 1 is provided with a negative pressure joint 5 and an irrigation joint 6, an irrigation tip and a negative pressure suction tip are installed in the suction and irrigation channel 3, the irrigation tip and the negative pressure suction tip may be positioned in the suction and irrigation channel 3 by using solidification glue, the negative pressure suction tip is in communication with the negative pressure joint 5 by passing through the tube portion and a negative pressure tube 7 of a cavity of the handle, and the irrigation tip is in communication with the irrigation joint 6 by passing through the tube portion and an irrigation tube 8 of the cavity of the handle; a roller-type negative pressure volume controller 11 for controlling a negative pressure value of the negative pressure tube 7 (which may be implemented by directly using a roller-type adjuster for adjusting a transfusion volume used in transfusion, where the negative pressure tube as a transfusion tube passes through the adjuster and contacts a roller, and the value of a negative pressure or on/off may be adjusted by adjusting a location of the roller) and a roller-type irrigation volume controller 12 for controlling an irrigation volume of the irrigation tube 8 (which may be implemented by directly using a roller-type adjuster for adjusting a transfusion volume used in transfusion, where the irrigation tube as a transfusion tube passes through the adjuster and contacts a roller, and the size of an irrigation volume or on/off may be adjusted by adjusting a location of the roller) are installed on the handle 1; an LED floodlight 9 is installed in the lighting channel 4, and electrically connected to a battery 13 installed in the handle 1 by using a conducting wire 10, and a push-button switch for controlling on/off of the LED floodlight 9 is installed on the handle 1.

During specific implementation, the structure in the suction and irrigation channel 3 may be autonomously designed according to needs, may be designed into a relatively sealed structure, an irrigation tube may directly stretch out from a sealed cavity, and moreover, a negative pressure suction tube is in communication with the sealed cavity, or one end of the negative pressure suction tube may be directly fixed to one end of the irrigation tube by using a sealant.

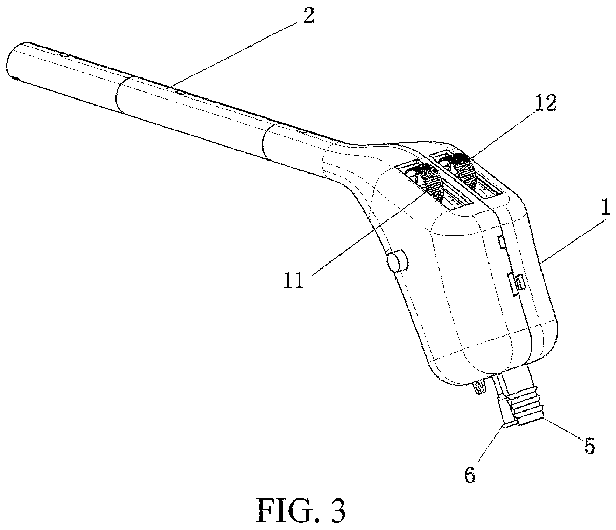

Moreover, during specific implementation, both the negative pressure tube and the suction tube may be of a structure of a soft tube or a soft tube plus a hard tube, but a part close to the roller-type negative pressure volume controller 11 and the roller-type irrigation volume controller 12 needs to be a soft tube so as to adjust the negative pressure value and the irrigation volume by adjusting the location of the roller. The roller-type negative pressure volume controller 11 and the roller-type irrigation volume controller 12 may be installed on the handle 1 abreast to adjust an upper surface of the roller protruding from the handle 1, as shown in FIG. 3. During use, as long as an operator pushes the roller to rotate, the corresponding negative pressure value or irrigation volume may be adjusted, and the use is very convenient.

### Embodiment 2

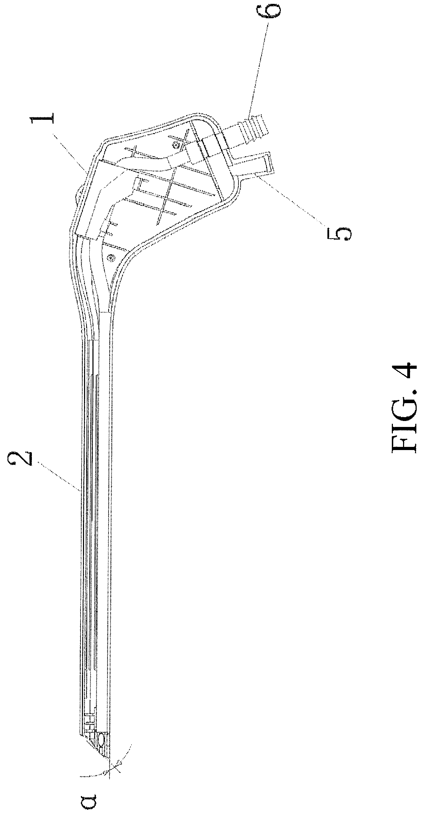

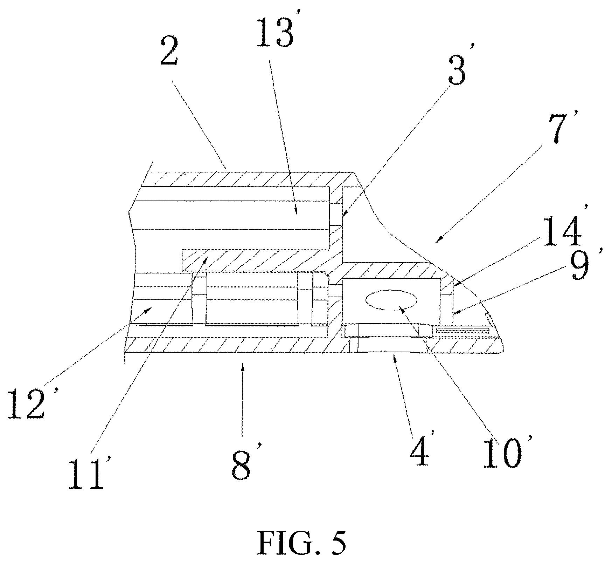

As shown in FIG. 4 to FIG. 5:
A multifunctional suction irrigator that performs suction from a bottom surface, and performs irrigation and suction on different end surfaces is formed by connecting a handle 1 and a tube portion 2 integrally, the handle 1 and the tube portion 2 may be injection molded by using a transparent material (or non-transparent material) to form a snap-fit casing structure, and when the handle 1 and the tube portion 2 are made of a transparent material, it is convenient to observe a blocking state of an internal channel during use. The handle 1 is connected to the tube portion 2 by using two smoothly transitional opposite arc segments, so as to further improve convenience of a surgical field and operations, where an opening of an arc segment close to the tube portion 2 is upward. For convenience of performing observation during an operation, in order to prevent the end tip of the irrigation tube from sheltering the visual field of irrigation, and for convenience of judging a bleeding position, during specific implementation, an end surface of the tube portion 2 should be designed into a slant shape of an inclined surface structure integrally formed by one or several inclined surfaces, and an angle α between the inclined surface and an axis of the tube portion should be designed to be less than 70 degrees, optimally about 30 degrees, as shown in FIG. 4 and FIG. 5. During specific implementation, for convenience of channel deployment and manufacturing, the inclined surface may be designed into a structure formed by two inclined surfaces whose angles are separately 60 degrees and 30 degrees, as shown in FIG. 5. Inside the end portion of the tube portion 2, the tube cavity of the end portion may be separated into a suction cavity 12' and an irrigation cavity 13' by using a separating plate 11', and a lighting apparatus may be further installed in the irrigation cavity 13'. A tail portion of the handle 1 is provided with a negative pressure joint 6 and an irrigation joint 5, the tube portion 2 is provided with a channel in communication with the negative pressure joint 6 and the irrigation joint 5, the channel (which may be a connection channel or a channel directly injection molded in the tube portion) is separately in communication with a negative pressure suction hole 4' and an irrigation outlet 3' corresponding to the end portion of the tube portion 2, the irrigation outlet 3' is located at an upper portion of an inclined surface 7' of the end portion of the tube portion 2, and the negative pressure suction hole 4' is mainly located on a bottom surface 8' adjacent to the inclined surface 7' of the end portion of the tube portion 2. That is, the axis of the negative pressure suction hole 4' and the axis of the irrigation outlet 3' are spatially intersected (spatially perpendicular or intersected), while the axis of the negative pressure suction hole 4' and the axis of the irrigation outlet 3' are spatially parallel in the prior art. A roller-type negative pressure volume controller for controlling a negative pressure value of the suction channel (the same as Embodiment 1, which may be implemented by directly using a roller-type adjuster for adjusting a transfusion volume used in transfusion, where the negative pressure tube as a transfusion tube passes through the adjuster and contacts a roller, and the value of a negative pressure or on/off may be adjusted by adjusting a location of the roller) and a roller-type irrigation volume controller for controlling an irrigation volume of the irrigation tube (the same as Embodiment 1, which may be also implemented by directly using a roller-type adjuster for adjusting a transfusion volume used in transfusion, where the irrigation tube as a transfusion tube passes through the adjuster and contacts a roller, and the size of an irrigation volume or on/off may be adjusted by adjusting a location of the roller) are installed on the handle 1; an LED floodlight may be installed at a side of the irrigation outlet of the end portion of the tube portion 2 and electrically connected to a battery installed in the handle 1 by using a conducting wire, and a push-button switch for controlling on/off of the LED floodlight is installed on the handle 1.

During specific implementation, besides that the main negative pressure suction hole 4' is disposed on the bottom surface 8' of the end portion of the tube portion 2, a negative pressure supplement suction hole 9' may be further disposed on the inclined surface 7', and to prevent an excessively large suction force of the negative pressure supplement suction hole 9' from affecting the bleeding position, a blocking plate 14' may be installed to the negative pressure supplement suction hole 9', as shown in FIG. 5. Moreover, several lateral negative pressure suction holes 10' may be further provided on hole walls of two sides or one side of the main negative pressure suction hole 4', the negative pressure suction hole 4', the negative pressure supplement suction hole 9' and the lateral negative pressure suction holes 10' should be in communication with each other, and a highest location of the negative pressure supplement suction hole 9' and the lateral negative pressure suction holes 10' does not exceed the height of a horizontal surface formed along a central axis of the tube portion 2, and the aperture of the supplement suction hole should be obviously less than the aperture of the suction hole 4'.

Moreover, during specific implementation, both the negative pressure tube and the suction tube may be of a structure of a soft tube or a soft tube plus a hard tube, but a part close to the roller-type negative pressure volume controller and the roller-type irrigation volume controller needs to be a soft tube so as to adjust the negative pressure value and the irrigation volume by adjusting the location of the roller. The roller-type negative pressure volume controller and the roller-type irrigation volume controller may be installed on the handle abreast to adjust an upper surface of the roller protruding from the handle, as shown in FIG. 4. During use, as long as an operator pushes the roller to rotate, the corresponding negative pressure value or irrigation volume may be adjusted, and the use is very convenient.

During use, the irrigation outlet of the present invention is aligned with the bleeding position to irrigate the bleeding position, and when flowing toward a low position, a liquid generated during the irrigation is just sucked by the negative pressure suction hole on the lower surface of the end portion of the tube portion. Because the negative pressure suction hole is not directly opposite to the bleeding position, the effect of the negative pressure suction force on the bleeding volume is fundamentally eliminated, and moreover, the large inclined surface of the tube portion increases a distance between an irrigation point and an suction point on one hand, and widens the surgical field on the other hand, so that a doctor is provided with higher proficiency during use.

Parts not involved in the present invention are all the same as those in the prior art or may be implemented by using the prior art.

## Claims

1. A multifunctional suction irrigator, comprising a handle (1) and a tube portion (2), wherein an end portion of one end, of the tube portion (2), not connected to the handle (1) is provided with a suction and irrigation channel (3) and a lighting channel (4), a tail portion of the handle (1) is provided with a negative pressure joint (5) and an irrigation joint (6), an irrigation tip and a negative pressure suction tip are installed in the suction and irrigation channel (3), the negative pressure suction tip is in communication with the negative pressure joint (5) by passing through the tube portion and a negative pressure tube (7) of a cavity of the handle, and the irrigation tip is in communication with the irrigation joint (6) by passing through the tube portion and an irrigation tube (8) of the cavity of the handle; a roller-type negative pressure volume controller (11) for controlling a negative pressure value of the negative pressure tube (7) and a roller-type irrigation volume controller (12) for controlling an irrigation volume of the irrigation tube (8) are installed on the handle (1); and an LED floodlight (9) is installed in the lighting channel (4), and electrically connected to a battery (13) installed in the handle (1) by using a conducting wire (10), and a push-button switch for controlling on/off of the LED floodlight (9) is installed on the handle (1).

2. The multifunctional suction irrigator according to claim 1, wherein an angle between an end surface of the tube portion (2) and an axis of the tube portion is less than 90 degrees so as to conveniently scrape and clean an operating position by using the end surface, and conveniently observe a bleeding position.

3. The multifunctional suction irrigator according to claim 1, wherein the irrigation tip and the negative pressure suction tip in the suction and irrigation channel (3) are positioned therein by using solidification glue, or fixed by using a blocking sheet on a tube wall.

4. The multifunctional suction irrigator according to claim 1, wherein an uppermost portion of the handle (1) is connected to the tube portion (2) by using two smoothly transitional opposite arc segments, so as to further improve convenience of a surgical field and operations, wherein an opening of an arc segment close to the tube portion (2) is upward.

5. The multifunctional suction irrigator according to claim 1, wherein the handle (1) and the tube portion are made of a transparent material, so as to conveniently observe a blocking state of an internal channel, or made of a non-transparent material.

6. A multifunctional suction irrigator, comprising a handle (1) and a tube portion (2), wherein an end portion of one end, of the tube portion (2), not connected to the handle (1) is provided with a suction and irrigation channel (3) and a lighting channel (4), a tail portion of the handle (1) is provided with a negative pressure joint (5) and an irrigation joint (6), an irrigation tip and a negative pressure suction tip are installed in the suction and irrigation channel (3), the negative pressure suction tip is in communication with the negative pressure joint (5) by passing through the tube portion and a negative pressure tube (7) of a cavity of the handle, and the irrigation tip is in communication with the irrigation joint (6) by passing through the tube portion and an irrigation tube (8) of the cavity of the handle; a roller-type negative pressure volume controller (11) for controlling a negative pressure value of the negative pressure tube (7) and a roller-type irrigation volume controller (12) for controlling an irrigation volume of the irrigation tube (8) are installed on the handle (1); and an irrigation outlet (3') in the suction and irrigation channel (3) is located at an upper portion of an inclined surface (7') of the end portion of the tube portion (2), and a negative pressure suction hole (4') of the negative pressure joint (5) is mainly located on a bottom surface (8') adjacent to the inclined surface (7') of the end portion of the tube portion (2).

7. The multifunctional suction irrigator according to claim 6, wherein a channel in the tube portion (2) is formed by a tube installed therein or a separate cavity formed in a tube; and the tube portion (2) is further provided with a lighting channel, and a lamp of the lighting channel is installed at a side of the irrigation outlet (3), or the tube portion (2) is provided with no lighting channel.

8. The multifunctional suction irrigator according to claim 6, wherein an adjustable switch for controlling a flow volume of a negative pressure channel and an adjustable switch for controlling a flow volume of an irrigation channel are disposed on the handle (1), and can control a water flow volume of irrigation and a flow volume of negative pressure suction respectively.

9. The multifunctional suction irrigator according to claim 6, wherein a negative pressure supplement suction hole (9') is also provided on the inclined surface (7') of the end portion of the tube portion (2), several lateral negative pressure suction holes (10') are provided on hole walls of two sides or one side of the negative pressure suction hole (4'), the negative pressure suction hole (4'), the negative pressure supplement suction hole (9') and the lateral negative pressure suction holes (10') are in communication with each other, and a highest location of the lateral negative pressure suction holes (10') does not exceed a horizontal surface of a central axis of the tube portion (2), and the aperture of the supplement suction hole should be obviously less than the aperture of the suction hole (4').

10. The multifunctional suction irrigator according to claim 6, wherein a slant angle α of the inclined surface (7') is less than 70 degrees; and the inclined surface (7') is formed by two segments of inclined surfaces with different slant angles, and a slant angle of an upper inclined surface is greater than a slant angle of a lower inclined surface.
